(19) Europäisches Patentamt
European Patent Office
Office européen des brevets

(11) Veröffentlichungsnummer: **0 038 465**
**A1**

(12) # EUROPÄISCHE PATENTANMELDUNG

(21) Anmeldenummer: **81102609.5**

(22) Anmeldetag: **07.04.81**

(51) Int. Cl.$^3$: **C 07 C 87/60**
C 07 C 85/11

(30) Priorität: **17.04.80 DE 3014716**

(43) Veröffentlichungstag der Anmeldung:
**28.10.81 Patentblatt 81/43**

(84) Benannte Vertragsstaaten:
**CH DE FR GB IT LI SE**

(71) Anmelder: **HOECHST AKTIENGESELLSCHAFT**
**Postfach 80 03 20**
**D-6230 Frankfurt/Main 80(DE)**

(72) Erfinder: **Franz, Raimund, Dr.**
**Johann-Strauss-Strasse 36**
**D-6233 Kelkheim (Taunus)(DE)**

(54) **Verfahren zur Herstellung von 2-Trifluormethylanilin.**

(57) 2-Trifluormethylanilin wird in ausgezeichneter Ausbeute hergestellt durch katalytische Reduktion und Dechlorierung des leicht zugänglichen 2-Trifluormethyl-4-chlor-nitrobenzols mit molekularem Wasserstoff in einem Reaktionsschritt in einem polaren Reaktionsmedium. Das Produkt ist hauptsächlich ein Zwischenprodukt, z.B. auf dem Farbstoffsektor.

EP 0 038 465 A1

Croydon Printing Company Ltd.

HOECHST AKTIENGESELLSCHAFT HOE 80/F 076 Dr. ME/Fr

## Verfahren zur Herstellung von 2-Trifluormethylanilin

2-Trifluormethylanilin ist ein wertvolles Zwischenprodukt auf verschiedenen Sachgebieten wie z.B. auf dem Farbstoffsektor zur Herstellung von Azofarbstoffen (vgl. DE-PS 12 27 585) etc.

Zur Herstellung des 2-Trifluormethylanilins sind verschiedene Methoden bekannt, die sich jedoch sämtlich für eine Durchführung in technischem Maßstab nur wenig eignen.

Eine ältere Methode (DE-PS 627 371) geht aus von o-Toluidin, dessen $NH_2$-Gruppe zunächst durch Überführung in ein o-Dicarbonsäureimid - insbesondere Phthalimid - geschützt wird. Dann wird die $CH_3$-Gruppe chloriert und fluoriert und schließlich wird die Imidstruktur wieder alkalisch oder sauer gespalten. Das Verfahren läßt sich durch folgendes Formelschema wiedergeben:

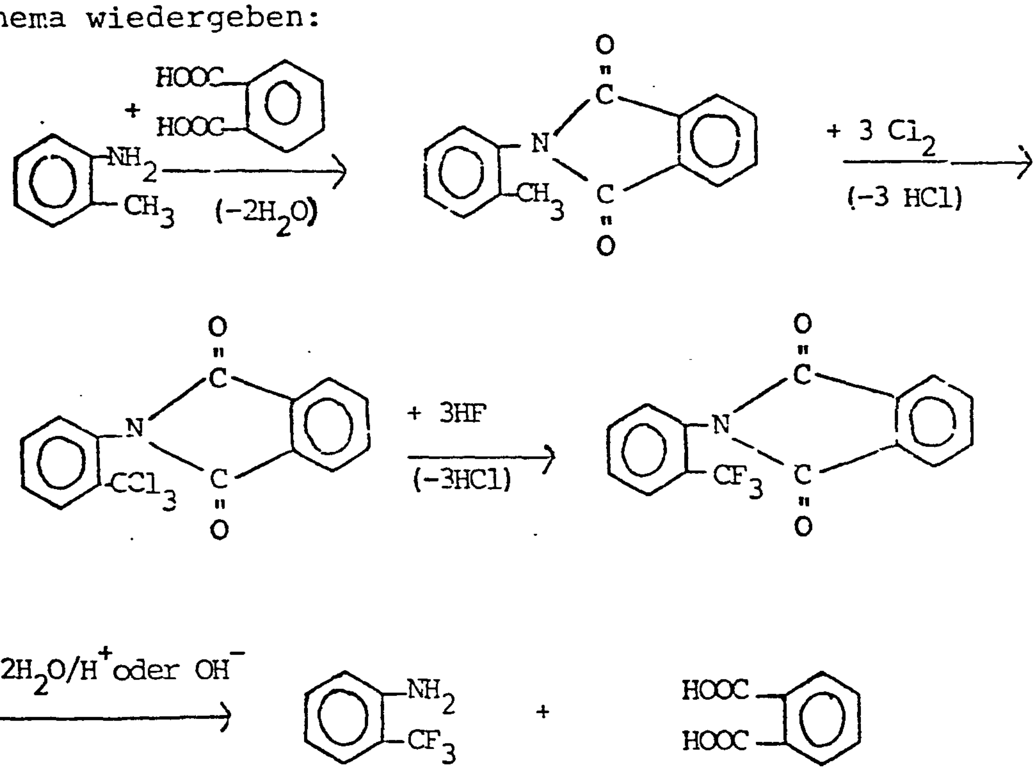


2-Trifluormethylanilin

Diese Methode ist aufwendig und kostspielig und hat deswegen keinen Eingang in die Technik gefunden.

Ein anderes, ebenfalls mehrstufiges, von japanischen Autoren (K.Fukui et al.) stammendes und in Chemical Abstracts Vol. 54 (1960), 4430e referiertes Verfahren geht aus von Trifluormethylbenzol (Benzotrifluorid); das Verfahren kann durch folgendes Formelschema wiedergegeben werden:

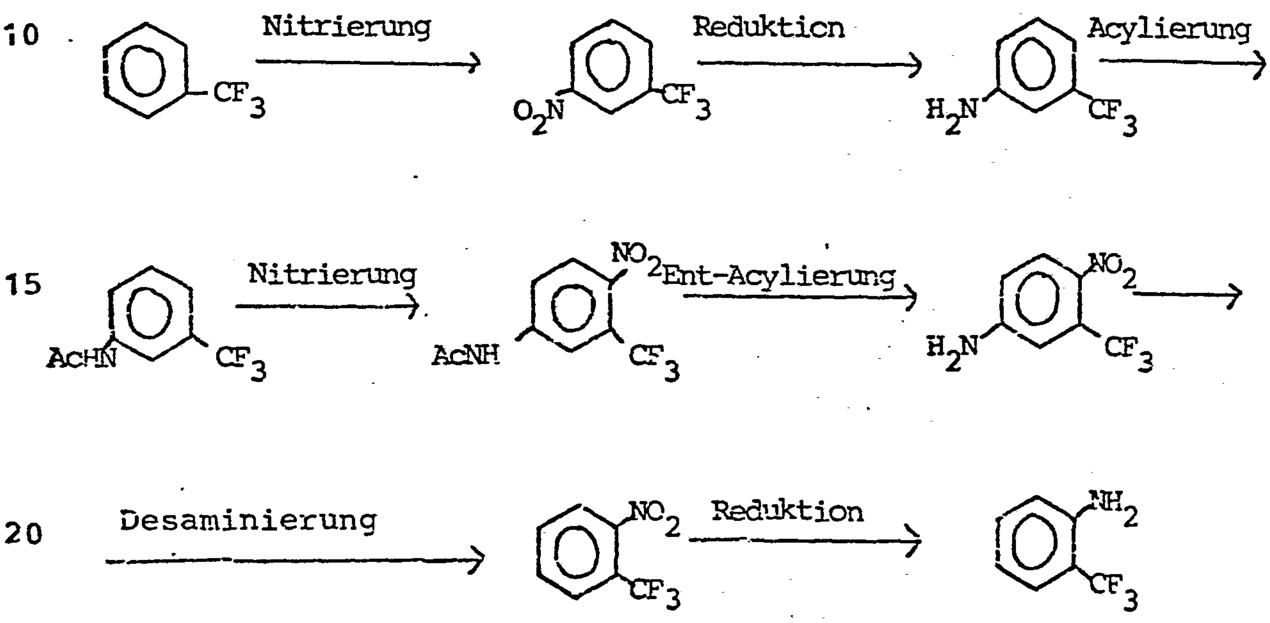


Die Reduktion der Nitroverbindungen (in der 2. sowie der letzten Reaktionsstufe) dürfte mit Fe/Säure erfolgen, da in dem Referat keine anderen Reduktionsmethoden erwähnt werden. Als Ausbeute der letzten Reaktionsstufe - also für die Reduktion des 2-Trifluormethyl-nitrobenzols zum 2-Trifluormethylanilin - sind 74 % d.Th. angegeben.

Wegen der Folge von 7 teilweise recht aufwendigen Reaktionsstufen ist eine wirtschaftliche Herstellung des 2-Trifluormethylanilins in technischem Maßstab nach diesem Verfahren

kaum möglich.

Der vielstufige und aufwendige Weg bis zum Ausgangsprodukt der letzten Reaktionsstufe - also bis zum 2-Trifluormethyl-nitrobenzol - ist bei diesem Verfahren wohl deswegen notwendig, weil sich das 2-Trifluormethyl-nitrobenzol nicht etwa durch direkte Nitrierung des Trifluormethylbenzols darstellen läßt; denn bei dieser direkten Nitrierung des Trifluormethylbenzols entsteht wegen der m-dirigierenden Wirkung der $CF_3$-Gruppe praktisch ausschließlich 3- (und nicht das hier erforderliche 2-) Trifluormethyl-nitrobenzol. 2-Trifluormethyl-nitrobenzol soll bei dieser Nitrierung nur spurenweise (weniger als 0,7 %) erhalten werden (vgl. Chemisches Zentralblatt 1921, Bd. III, S. 32).

Wenn das 2-Trifluormethyl-nitrobenzol - auf welche Weise auch immer - hergestellt ist, kann dessen Reduktion zu 2-Trifluormethylanilin mit fast quantitativer Ausbeute auch durch katalytische Hydrierung in wässriger Phase mittels Nickel-Katalysatoren erfolgen, wenn man durch Zusatz von Sauerstoffsäuren des 3- oder 5-wertigen Phosphors, des Bors, der Kohlensäure oder niedermolekularer Carbonsäuren und ausreichend starker Basen bzw. entsprechender Salze einen pH-Bereich von 6 bis 7,5 einstellt und die Zusätze so dosiert, daß dieser Bereich im Laufe der Reaktion nicht unterschritten wird (NL-OS 73 11 193 - HOE 72/F 257 K).

Es ist weiterhin auch noch bekannt, daß 2-Trifluormethylanilin in etwa 60 %iger Ausbeute durch katalytische Dechlorierung von 2-Trifluormethyl-4-chlor-anilin - d.i. also der Verbindung der Formel

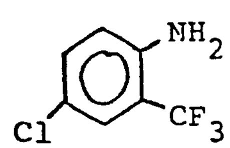

entsteht (DE-OS 25 40 740, Bsp. 2). Die katalytische Dechlorierung wird hier in der Siedehitze mit einem Salz der Ameisensäure in Gegenwart eines Hydrierkatalysators (in besagtem Beispiel 2: Palladium auf Kohle) und eines oberflächenaktivem Mittels (vorzugsweise eines Phasentransferkatalysators) in alkalischem Medium durchgeführt; sie stellt einen Spezialfall bzw. eine Abwandlung der in Houben-Weyl "Methoden der Organischen Chemie" Bd. V/4 "Halogenverbindungen" (1960), S. 769 bis 770 beschriebenen allgemeinen Methoden zum Austausch von Halogen gegen Wasserstoff in aromatischen Halogenverbindungen mittels katalytisch erregtem Wasserstoff in alkalischem Medium dar.

In der DE-OS heißt es auch, daß bei der dort offenbarten Reaktion für den Fall, daß in den zu dehalogenierenden Verbindungen außer den Halogenatomen noch reduzierbare Gruppen wie z.B. Nitrogruppen vorliegen, diese während der Reaktion reduziert werden, was in Beispiel 1 der DE-OS mit der Herstellung von Anilin (in 83 %iger Ausbeute) aus 2-Chlornitrobenzol belegt wird. Die Nacharbeitung dieses Beispiels mit 2-Trifluormethyl-4-chlor-nitrobenzol (anstelle des 2-Chlor-nitrobenzols) als Ausgangsmaterial ergab jedoch hauptsächlich 2,2'-Bis-(trifluormethyl)benzidin (etwa 50 % d.Th.) und nur etwa 30 % d.Th. des an sich erwarteten 2-Trifluormethylanilins.

Die DE-OS 25 40 740 konnte somit kaum dazu anregen, weitere Versuche der katalytischen Reduktion und Dehalogenierung von 2-Trifluormethyl-halogen-nitrobenzolen zu unternehmen, wenn man 2-Trifluoranilin nicht nur als Nebenprodukt, sondern als wesentliches Hauptprodukt in möglichst quantitativer Ausbeute erhalten wollte.

In dem Bestreben, die bekannten Verfahren zur Herstellung von 2-Trifluormethylanilin zu verbessern oder ein neues verbessertes, auch in technischem Maßstab wirtschaftlich durchführbares Verfahren zu entwickeln, wurde nun gefunden, daß sich dieses Ziel erreichen läßt, wenn man das gut zugängliche 2-Trifluormethyl-4-chlor-nitrobenzol als Ausgangsmaterial verwendet und dieses in einem Reaktionsschritt in einem polaren Reaktionsmedium mit molekularem Wasserstoff katalytisch reduziert und dechloriert.

Erfindungsgegenstand ist somit ein Verfahren zur Herstellung von 2-Trifluormethylanilin durch reduktive Behandlung einer 2-Trifluormethyl-nitrobenzol-Ausgangsverbindung, das dadurch gekennzeichnet ist, daß man 2-Trifluormethyl-4-chlor-nitrobenzol in einem Reaktionsschritt in einem polaren Reaktionsmedium mit molekularem Wasserstoff katalytisch reduziert und dechloriert.

Im Hinblick auf die Tatsache, daß die in der DE-OS 25 40 740 beschriebene Verfahrensweise beim Einsatz des 2-Trifluormethyl-4-chlor-nitrobenzols 2-Trifluormethylanilin nur in sehr unbefriedigender Ausbeute (etwa 30 % d.Th.) lieferte, lag der Schluß nahe, daß die Ursache hierfür in erster Linie in der speziellen Natur der eingesetzten Ausgangsverbindung zu suchen ist und daß weitere Versuche einer reduktiven und dechlorierenden Behandlung von 2-Trifluormethyl-4-chlornitrobenzol mit dem Ziel der Herstellung von 2-Trifluormethylanilin in guten bis sehr guten Ausbeuten kaum sinnvoll sind. Es war daher außerordentlich überraschend, daß es durch das erfindungsgemäße Verfahren gelang, das gut zugängliche 2-Trifluormethyl-4-chlor-nitrobenzol zu dem gewünschten 2-Trifluoranilin in Ausbeuten bis zu etwa 95 % d.Th. katalytisch mit molekularem Wasserstoff zu reduzieren und dechlorieren.

Die Ausgangsverbindung für das Verfahren - das 2-Trifluormethyl-4-chlor-nitrobenzol - ist in ziemlich glatter Reaktion gemäß DE-PS 637 318 durch Chlorierung und Nitrierung von Trifluormethylbenzol erhältlich:

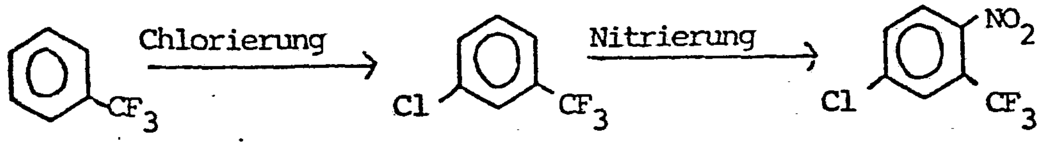


Als polare Reaktionsmedien kommen im Prinzip alle möglichen polaren Lösungs- und Verdünnungsmittel in Frage, welche unter den angewandten Reaktionsbedingungen mit den Ausgangs- und Endstoffen nicht reagieren, wie z.B. Wasser, niedere Alkohole (Methanol, Äthanol, Propanole, Butanole), Äther (Dioxan, Diäthylenglykol-diäthyläther etc.) etc. Bevorzugte polare Reaktionsmedien sind Wasser und die $C_1$-$C_3$-Alkohole, insbesondere Methanol. Es kann sowohl eines der genannten Lösungs- und Verdünnungsmittel als auch eine Mischung daraus verwendet werden.

Das Mengenverhältnis der Ausgangsverbindung (2-Trifluormethyl-4-chlor-nitrobenzol) zum polaren Reaktionsmedium ist nicht kritisch und richtet sich hauptsächlich nach den gegebenen Möglichkeiten der mechanischen Rührung und Durchmischung der im Reaktionsansatz vorhandenen festen (Katalysator), flüssigen (Substrat, Produkt, Medium) und gasförmigen ($H_2$) Phasen.

Als Katalysatoren können praktisch alle üblichen Hydrierkatalysatoren - z.B. Pd-, Pt-, Ni- etc. Katalysatoren - Verwendung finden. Bevorzugte Katalysatoren sind Pd auf Kohle sowie (Raney-)Nickel.

Die verwendete Katalysatormenge richtet sich zweckmäßig nach der Aktivität des eingesetzten Katalysators, ist aber im Prinzip nicht kritisch.

Das erfindungsgemäße Verfahren verläuft nach folgender Summengleichung:

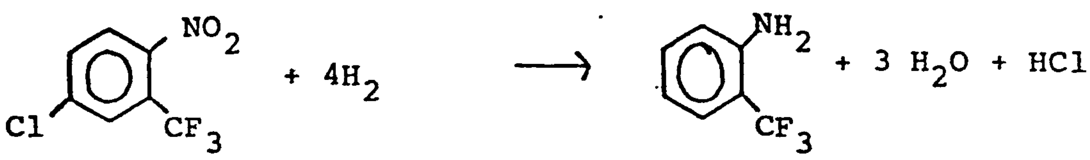

Wie aus dieser Gleichung ersichtlich, entsteht bei der Reaktion eine nicht unwesentliche Menge Chlorwasserstoff. Es ist im Prinzip nicht erforderlich, den Chlorwasserstoff durch basische Mittel zu binden, doch werden höhere Ausbeuten durch einen entsprechenden Basenzusatz erhalten. Der Basenzusatz, der zu Anfang, während oder vorzugsweise gegen Ende der Hydrierungsreaktion erfolgen kann, verhindert außerdem eine Korrosion oder zumindest Inaktivierung des Katalysators durch den Chlorwasserstoff und erlaubt eine erleichterte Aufarbeitung des Reaktionsgemisches.

Als Basen kommen im Prinzip alle möglichen Chlorwasserstoffbindenden basischen Verbindungen infrage; bevorzugt sind jedoch die Alkalihydroxide (davon insbesondere NaOH), Ammoniak und niedere aliphatische Amine (bis zu insgesamt 10 C-Atomen, insbesondere Triäthylamin).

Die Reaktionstemperaturen können im Prinzip zwischen etwa 0 und etwa 150°C liegen. Bevorzugt sind Temperaturen zwischen etwa 10 und etwa 80°C, insbesondere zwischen etwa 20 und etwa 30°C.

Außerdem kann die Reaktion sowohl drucklos (z.B. in Glasgefäßen) als auch unter Wasserstoff-Überdruck (z.B. in metallenen Druckgefäßen) durchgeführt werden.

Die Aufarbeitung des Reaktionsansatzes erfolgt auf übliche Weise, d.i. im vorliegenden Fall also vorzugsweise (nach Abfiltration vom Katalysator und gegebenenfalls Phasentrennung) durch Destillation. Das Reaktionsmedium kann, wenn gewünscht mit nur geringen Verlusten immer wieder zurückgewonnen und für einen neuen Ansatz eingesetzt werden. Die Ausbeute an 2-Trifluormethylanilin beträgt bis zu etwa 95 % d.Th., bezogen auf das Ausgangsprodukt 2-Trifluormethyl-4-chlor-nitrobenzol.

Das Verfahren zeichnet sich vor allem durch den Einsatz einer relativ einfach und gut zugänglichen Ausgangsverbindung, die außerordentlich einfache und wirtschaftliche Durchführung in nur einem Reaktionsschritt, die problemlose Aufarbeitung und eine ausgezeichnete Ausbeute an dem gewünschten Endprodukt aus. Wenn man nach dem Stand der Technik das 2-Trifluormethyl-4-chlor-nitrobenzol zuerst mit Fe/HCl zu dem 2-Trifluormethyl-4-chlor-anilin reduziert [Chemical Abstracts Vol. 50 (1956), 488b-c; Ausbeute 85-87 %] und dieses dann mit Na-Formiat/Pd/C/NaOH/Phasentransferkatalysator dechloriert (nach DE-OS 25 40 740, Beispiel 2; Ausbeute 60 %), kommt man auf einem aufwendigeren Weg auf eine Ausbeute von nur 51 bis 52 % d.Th. an 2-Trifluormethylanilin, bezogen auf 2-Trifluormethyl-4-chlor-nitrobenzol.

Die Erfindung stellt somit einen erheblichen Fortschritt dar.

Die folgenden Beispiele sollen nun der näheren Erläuterung der Erfindung dienen. Nach den Erfindungsbeispielen 1 bis 7 folgt ein Vergleichsbeispiel (Nacharbeitung des Beispiels 1 der DE-OS 25 40 740 mit 2-Trifluormethyl-4-chlor-nitrobenzol als Ausgangsmaterial).

Beispiel 1

In einem 1 l-Rührkolben mit Magnetrührverschluß wurde eine

Lösung von 45 g 2-Trifluormethyl-4-chlor-nitrobenzol in 200 ml Methanol in Gegenwart von 2 g einer 50-%igen wässrigen Suspension von Palladium auf Aktivkohle (5 % Pd) unter starkem Rühren mit molekularem Wasserstoff bei geringem Überdruck (max. 0,2 bar) hydriert. Die Reaktionswärme wurde durch Kühlen im Eiswasserbad abgeführt und die Innentemperatur damit auf 20 - 25°C gehalten. Nach Abklingen der raschen Gasaufnahme wurde eine Lösung von 8 g Natriumhydroxid in 50 ml Methanol zugetropft und der Ansatz zu Ende hydriert. Der Katalysator wurde dann abgesaugt, mit wenig Methanol nachgewaschen und die vereinigten Filtrate durch eine 40 cm lange Vigreux-Kolonne bei Normaldruck destilliert bis im Destillationssumpf Phasentrennung eintrat. Der aus einer wässrigen und einer organischen Phase bestehende Sumpf wurde nun dampfdestilliert. Die organische Phase des Destillates wog 31 g und bestand nach Gaschromatogramm zu 98,3 % aus 2-Trifluormethylanilin, was einer Ausbeute von 95 % d.Th. entspricht.

Beispiel 2

90 g 3-Trifluormethyl-4-chlor-nitrobenzol und 42 g Triethylamin wurden in 400 ml Methanol gelöst und 10 g eines Palladium-Kohle-Katalysators zugegeben. Dieses Gemisch wurde in einer Schüttelente mit molekularem Wasserstoff umgesetzt. Es wurden (umgerechnet auf Normalbedingungen) 36 l Wasserstoff aufgenommen. Nach Reaktionsende wurde der Katalysator abgesaugt und mit Methanol nachgewaschen. Das Filtrat wurde unter Zuhilfenahme einer Kolonne eingeengt, bis es sich in zwei flüssige Phasen getrennt hatte. Nach Zusatz einiger ml konzentrierter Natronlauge wurde die organische Phase abgetrennt und im Vakuum destilliert. Die Ausbeute betrug 50 g 2-Trifluormethylanilin (78 % d.Th.) vom Sdp. 60°C/10mbar.

Beispiel 3

In einem 500 ml-Kolben mit Magnetrührverschluß wurde ein Gemisch aus 45 g 2-Trifluormethyl-4-chlor-nitrobenzol,

100 ml Wasser und 2 g einer 50 %igen wässrigen Suspension von Palladium auf Aktivkohle im Lauf von 8 Stunden unter starkem Rühren bei 75°C hydriert. Nach Aufnahme von etwa 80 % der theoretischen Menge des Wasserstoffs wurde eine Lösung von 8 g Natriumhydroxid in 30 ml Wasser zugetropft und die Reaktion zu Ende geführt. Das nach scharfem Absaugen des Katalysators erhaltene, aus zwei flüssigen Phasen bestehende Filtrat wurde mit Wasserdampf destilliert. Die organische Phase des Destillats wog 26 g (81 % d.Th.) und bestand laut Gaschromatogramm zu 98,7 % aus 2-Trifluormethylanilin.

Beispiel 4

In einem Einliterkolben mit Magnetrührverschluß wurde eine Lösung von 113 g 2-Trifluormethyl-4-chlor-nitrobenzol in 180 ml der Methanolphase eines gleichen, vorhergenden Ansatzes in Gegenwart von 5 g Palladium-Kohle-Katalysator mit molekularem Wasserstoff bei max. 0.2 bar Überdruck hydriert. Der Katalysator bestand zu 10 % aus frischem, zu 90 % aus z.T. mehrfach für die gleiche Reaktion gebrauchtem Material. Durch gelegentliche Außenkühlung wurde erreicht, daß die autogene Reaktionstemperatur 50°C nicht überstieg. Nach Aufnahme von ca. 30 l Wasserstoff wurden 60 g 33 %ige Natronlauge zugetropft und die Reaktion zu Ende geführt. Nun wurde der Katalysator abgesaugt und das Filtrat in einem Dünnschichtverdampfer vom größten Teil des Methanols befreit. Die im Sumpfprodukt enthaltene organische Phase wog 68 g, zeigte ein Infrarotspektrum, welches mit dem von authentischem Material genau identisch war und enthielt nach Gaschromatogramm 9,2 % Methanol sowie 90 % 2-Trifluormethylanilin, was einer Ausbeute von 77,2 % d.Th. entspricht.

Beispiel 5

Eine Lösung von 45 g 2-Trifluormethyl-4-chlor-nitrobenzol in 200 ml Methanol wurde in Gegenwart von 25 g frisch hergestelltem Raney-Nickel in einer Schüttelente bei Raumtemperatur hydriert. Nach Aufnahme von etwa 10 l Wasserstoff wurde eine Lösung von 8 g Natriumhydroxid in 100 ml Methanol zugegeben und die Reaktion zu Ende geführt. Dann wurde der Katalysator abgesaugt, vom Filtrat das Methanol abfraktioniert, der Destillationsrückstand mit Methylenchlorid extrahiert, die Extraktionslösung getrocknet und im Vakuum destilliert. Die Ausbeute betrug 20 g (62,5 %d.Th.) vom Sdp. 60°C bei 10 mbar.

Beispiel 6

In einem 500 ml-Kolben mit Magnetrührverschluß wurde eine Lösung von 45 g 2-Trifluormethyl-4-chlor-nitrobenzol in 100 ml n-Propanol in Gegenwart von 4 g einer 50 %igen wässrigen Suspension eines Palladium-Kohle-Katalysators bei leichtem Überdruck hydriert. Nach Aufnahme der Hälfte der theoretisch errechenbaren Wasserstoffmenge wurden 25 g 33 %ige Natronlauge im Lauf einer Stunde zugetropft und die Reaktion zu Ende geführt. Das Gesamtgewicht der Reaktionslösung betrug nach dem Absaugen vom Katalysator 140 g. Sie enthielt nach Gaschromatogramm 18 % 2-Trifluormethylanilin, was einer Ausbeute von 78,8 % d.Th. entspricht.

Beispiel 7

In einem 500 ml-Kolben mit Magnetrührverschluß wurde eine Lösung von 45 g 2-Trifluormethyl-4-chlor-nitrobenzol in 100 ml Methanol mit 15 g 25 %iger wässriger Ammoniaklösung versetzt und in Gegenwart von 4 g einer 50 %igen wässrigen Suspension eines Palladium-Aktivkohle-Katalysators (5 % Pd) mit molekularem Wasserstoff hydriert. Die Reaktion verlief während der ersten drei Stunden sehr rasch und exotherm. Nach weiteren 2,5 Stunden wurde vom Katalysator abgesaugt, mit wenig Methanol nachgewaschen und die vereinigten Filtrate in einem Dünnschichtverdampfer destilliert. Die organische Phase des Destillationssumpfes (23 g) enthielt nach Gaschromatogramm 2-Trifluormethylanilin zu 93 %, das

Methanol-Destillat (111 g) zu 8 %. Hieraus ergibt sich eine Gesamtausbeute von 94,7 % d.Th.

Vergleichsbeispiel

(Nacharbeitung von Beispiel 1 der DE-OS 25 40 740 mit 2-Trifluormethyl-4-chlor-nitrobenzol als Ausgangsmaterial)

Zu einer Lösung von 75 g Natriumformiat in 150 ml Wasser wurden 12,6 g 33 %ige Natronlauge, 1 g Benzyltriethylammoniumchlorid, 6 g einer 50 %igen wässrigen Suspension eines Palladium-Kohle-Katalysators (3 % Pd) sowie 22,6 g (0,1 Mol) 2-Trifluormethyl-4-chlor-nitrobenzol gegeben. Das Gemisch wurde stark gerührt, 4 Stunden am Rückfluß erhitzt und anschließend mit Wasserdampf destilliert. Im Destillat fanden sich 5 g 2-Trifluormethylanilin (ca. 31 % d.Th.) als flüssige schwere Phase, sowie ein im Wasser suspendierter Feststoff in Form farbloser Nadeln, von dem die Hauptmenge aus dem ursprünglichen Reaktionsgemisch durch Absaugen des Katalysators, Waschen mit Methanol und Fällen mit verd. Lauge gewonnen werden konnte. Ausbeute: 8 g 2,2'-Bis(trifluormethyl)-benzidin ( 50 % d.Th.) vom Schmelzpunkt 111°C. Der Literatur-Schmelzpunkt liegt bei 112 - 113°C (Y. Maki et al., Nippon Kagaku Kaishi 1972 (3), S. 676).

1. Verfahren zur Herstellung von 2-Trifluormethylanilin durch reduktive Behandlung einer 2-Trifluormethyl-nitrobenzol-Ausgangsverbindung, dadurch gekennzeichnet, daß man 2-Trifluormethyl-4-chlor-nitrobenzol in einem Reaktionsschritt in einem polaren Reaktionsmedium mit molekularem Wasserstoff katalytisch reduziert und dechloriert.

2. Verfahren nach Anspruch 1, dadurch gekennzeichnet, daß man als polares Reaktionsmedium Wasser und/oder mindestens einen $C_1$-$C_3$-Alkohol, vorzugsweise Methanol, verwendet.

3. Verfahren nach den Ansprüchen 1 bis 2, dadurch gekennzeichnet, daß man als Katalysator Palladium (auf Kohle) oder Raney-Nickel verwendet.

4. Verfahren nach den Ansprüchen 1 - 3, dadurch gekennzeichnet, daß man zur Bindung des bei der Dechlorierung freiwerdenden Chlorwasserstoffs eine Base zusetzt.

5. Verfahren nach Anspruch 4, dadurch gekennzeichnet, daß man als Base ein Alkalihydroxid, Ammoniak oder ein niederes aliphatisches Amin verwendet.

6. Verfahren nach den Ansprüchen 1 bis 5, dadurch gekennzeichnet, daß man die katalytische Reduktion und Dechlorierung bei Temperaturen zwischen etwa 10 und etwa 80°C, insbesondere zwischen etwa 20 und etwa 30°C, durchführt.

0038465

Europäisches Patentamt

**EUROPÄISCHER RECHERCHENBERICHT**

Nummer der Anmeldung

EP 81 10 2609

**EINSCHLÄGIGE DOKUMENTE**

| Kategorie | Kennzeichnung des Dokuments mit Angabe, soweit erforderlich, der maßgeblichen Teile | betrifft Anspruch |
|---|---|---|
| X | LU - A - 62 645 (BAYER)<br>* Ansprüche; Beispiel 4 *<br>-- | 1-6 |
| | EP - A - 0 001 825 (MERCK)<br>* Ansprüche; Beispiel 8; Seiten 4,5 *<br>-- | 1 |
| D | FR - A - 2 285 355 (I.C.I.)<br>* Ansprüche; Beispiel 2 *<br>& DE - A - 2 540 740<br>---- | 1,3-5 |

KLASSIFIKATION DER ANMELDUNG (Int. Cl.³)

C 07 C 87/60
85/11

RECHERCHIERTE SACHGEBIETE (Int. Cl.³)

C 07 C 87/60
85/11

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 23-07-1981 | MOREAU |